# EUROPEAN PATENT SPECIFICATION

(11) **EP 2 932 929 B1**
(45) Date of publication and mention of the grant of the patent: **08.02.2017**
(21) Application number: 14164692.7
(22) Date of filing: 15.04.2014
(51) Int. Cl.: A61B 17/86, A61B 17/88

(54) **A screw element for use in spinal, orthopedic or trauma surgery and a system of such a screw element and a screw driver adapted thereto**
Schraubelement zur Verwendung in der spinalen, orthopädischen oder traumatologischen Chirurgie und System mit solch einem Schraubelement und daran angepassten Schraubendreher
Élément de vis destiné à être utilisé dans la colonne vertébrale, en chirurgie des accidents ou orthopédique, système d'un tel élément de vis et tournevis adapté à celui-ci

(43) Date of publication of application: 21.10.2015
(73) Proprietor: Biedermann Technologies GmbH & Co. KG, 78166 Donaueschingen (DE)
(72) Inventor: Biedermann, Lutz, 78048 VS-Villingen (DE); Dandanopoulous, Dimosthenis, 78056 VS-Schwenningen (DE); Matthis, Wilfried, 79367 Weisweil (DE)
(74) Representative: Prüfer & Partner mbB Patentanwälte · Rechtsanwälte

(56) References cited:
- WO-A1-2014/035764
- US-A1- 2003 113 690
- US-A1- 2011 077 693
- US-A1- 2011 306 984

## Description

The invention relates to a screw element for use in spinal, orthopedic or trauma surgery and to a screw driver adapted to the screw element. The screw element includes a drive portion for engagement with a screw driver wherein the drive portion comprises drive grooves for engagement with corresponding engagement protrusions of the screw driver and guide grooves that are configured to guide the engagement protrusions of the screw driver into the drive grooves. The screw element can be used in particular in minimally invasive surgery and other procedures such as minimal access surgery where the view onto and/or the access to the operation site is reduced.

In particular in spinal surgery, surgical techniques have become known that include a step of mounting a receiving part of a polyaxial pedicle screw onto the screw element *in situ* after placement of the screw element into the pedicle of a vertebra. For example, in a surgical technique known under the name interpedicular minimal access surgery a small incision is made and several motion segments of the spine are treated through the small incision. First, the screw elements with ball-shaped heads are inserted into the pedicles using an instrument that holds the screw elements so that they cannot become accidentally detached from the instrument and that further acts as a screw driver to insert the screw elements. The screw elements are inserted into the pedicles to a certain depth that might not be the final depth for the screw element. In a second step the actual insertion depth is determined with the aid of, for example an X-ray image, and thereafter the insertion depth is precisely adjusted to a desired depth on the basis of such an X-ray image. Finally, the receiving parts are mounted onto the screw elements and a stabilization rod is connected to the receiving parts.

WO 2014/035764 A1 describes a surgical implant driver with an engagement portion. The engagement portion includes a first surface with a drive interface engagable with a first surface of an implant fastener and a second surface with at least a retention interface engagable with a second surface of the implant fastener.

US 2003/0113690 A1 describes a dental implant-abutment interface and assembly comprising an implant, an abutment removably attachable to the implant, and a screw receivable within an axial threaded bore common to both the implant and abutment. The implant includes a plurality of axially circular recesses and the abutment includes prongs projecting distally from a radial plane, whereat each of the prongs is proportioned for engagement with a respective one of said axial recesses of the implant.

In the step of exactly adjusting the insertion depth of the screw elements, a screw driver is used that is configured to engage a drive portion of the screw element. With the known screw elements and drivers there might sometimes be a problem to locate the drive portion of the screw element if the sight onto the operation site is restricted or if the respective screw element is not visible at all.

It is the object of the invention to provide a screw element and a system of a screw element and a corresponding screw driver adapted thereto that allows to perform the adjustment of an insertion depth in a quick and safe manner.

The object is solved by a screw element according to claim 1 and by a system comprising such a screw element and a screw driver adapted to the drive portion of the screw element according to claim 13. Further developments are given in the dependent claims.

The screw element permits to more easily locate the drive portion with the screw driver. In addition, the insertion of the engagement portion of the screw driver into the drive portion of the screw element is facilitated. Therefore, also in a case where the screw driver is applied in a slightly inclined manner, the design of the drive portion of the screw element effects an alignment of the screw axis and the axis of the screw driver. The screw driver is an instrument that is free from any complex functions and therefore permits an easy and convenient handling.

The screw element may be a bone screw with a head that comprises the drive portion. However, it may also be a set screw that is used as a locking element in the receiving part of a polyaxial bone screw or in a bone plate. More generally, the screw element may be used in cases with poor or no sight onto the operation site where it is required to adjust the position of a screw that has already been placed.

Further features and advantages of the invention will become apparent from the description of embodiments by means of the accompanying drawings. In the drawings:
- Fig. 1: shows a perspective view of a screw element according to a first embodiment with a portion of a screw driver adapted to a drive portion of the screw element.
- Fig. 2: shows an enlarged view of a detail of Fig. 1.
- Fig. 3: shows a perspective view from the top onto the screw element of Figs. 1 and 2.
- Fig. 4: shows a top view onto the screw element of Figs. 1 to 3.
- Fig. 5: shows a cross-sectional view of the screw element of Figs. 1 to 4 along line A-A in Fig. 4.
- Fig. 6: shows a side view onto a screw driver with an engagement portion adapted to the drive portion of the screw element of Figs. 1 to 5.
- Fig. 7: shows an enlarged perspective view onto the engagement portion of the screw driver of Fig. 6.
- Fig. 8: shows a perspective view from the top onto a screw element according to a modified embodiment.
- Fig. 9: shows an enlarged perspective view of a step of using the screw element and the screw driver according to the first embodiment.
- Fig. 10: shows a perspective view of a step of adjusting the insertion depth of the screw element.
- Figs. 11a) to 11c): show cross-sectional views of steps of engaging the drive portion of the screw element according to the first embodiment with the engagement portion of the screw driver.
- Fig. 12: shows a perspective view of a second embodiment of the screw element in connection with a polyaxial bone anchor.

- Fig. 13: shows a perspective view of a further application of the screw element in connection with a bone plate.

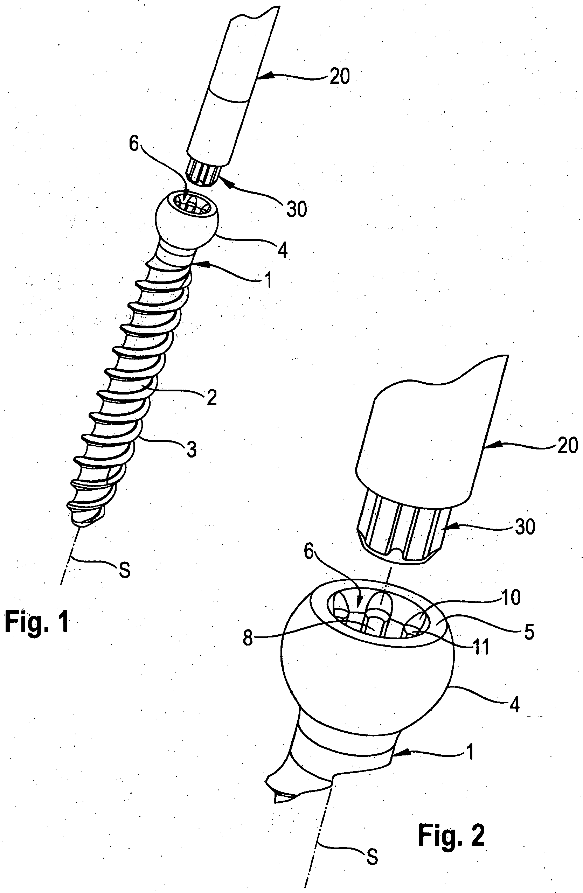

Referring to Figs. 1 to 5, a screw element 1 according to a first embodiment comprises a shank 2 with a bone thread 3 in at least a portion of the shank 2 and a head 4. The shank 2 is configured to be inserted into a bone or in particular into a pedicle of a vertebra. A screw axis S is defined by the axis of the bone thread 3. The head 4 has the shape of a segment of a sphere and a free end 5 on a side that is opposite to the shank 2. The bone thread 3 defines a screw axis S. At the free end 5, a drive portion 6 that is configured to be engaged with an engagement portion of a driver is provided. The drive portion 6 will be explained more in detail below.

A system of the screw element 1 with the drive portion 6 and a screw driver includes a screw driver 20 that has an engagement portion 30 adapted for engagement with the drive portion 6 of the screw element 1.

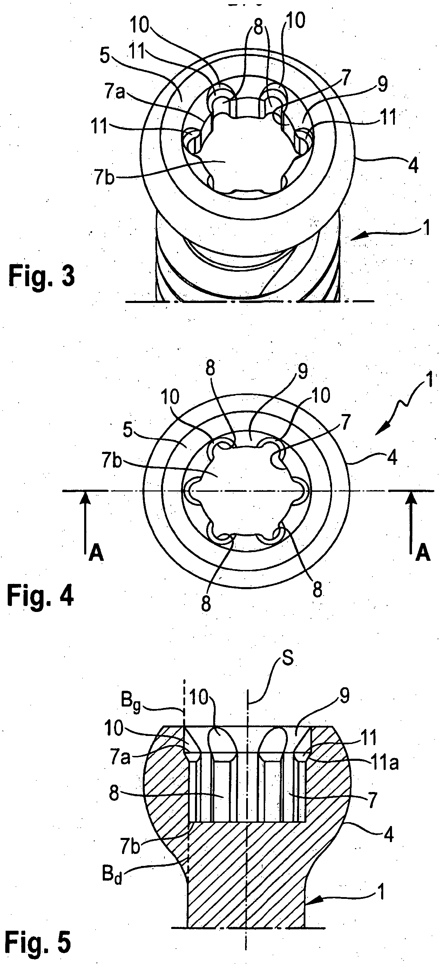

As depicted in particular in Figs. 3 to 5, the drive portion 6 of the screw element 1 comprises a first recess 7 that is located at a distance from the free end 5 and that has a substantially cylindrical main contour with a main inner diameter and with the cylinder axis being the screw axis S. In the cylinder wall of the substantially cylindrical first recess 7, a plurality of longitudinal drive grooves 8 are formed. The drive grooves 8 each comprise a bottom line B_{d} that is parallel to the screw axis S (see Fig. 5). A cross-section of the drive grooves 8 in a plane perpendicular to the screw axis S is substantially circular segment-shaped. The drive grooves 8 are arranged in a top view, as seen in Fig. 4, in a star-like manner. In a specific embodiment, the first recess 7 together with the drive grooves 8 forms a torx-shaped drive structure that is configured to be engaged by a torx-shaped engagement portion of the screw driver. The upper end 7a of the recess 7 is positioned at a distance from the free end 5 of the head 4. An axial depth from the end 7a to the lower end 7b of the first recess 7 corresponds substantially to a depth of usual drive recesses for screw elements of this type. In other words, the size of the first recess 7 with the drive grooves 8 is sufficient for applying the necessary torque to the screw element 1.

Between the first recess 7 and the free end 5 there is a second recess 9 that conically tapers from the free end 5 towards the first recess 7. More in detail, the lower diameter of the second recess 9 is slightly larger than the main diameter of the first recess 7 and the upper diameter of the second recess 9 is greater than the lower diameter. The depth of the second recess 9 in the axial direction corresponds to approximately one fifth to one third of the depth of the first recess 7, preferably between one fourth and one third of the depth of the first recess 7. The second recess 9 provides an enlarged bevelled surface that facilitates the insertion of the engagement portion 30 of the screw driver 20.

A plurality of guide grooves 10 are provided in the wall defining the second recess 9 at positions corresponding to the positions of the drive grooves 8 in the first recess 7. Each of the guide grooves 10 comprises a longitudinal bottom line Bg that is parallel to the screw axis S and also parallel to the bottom line B_{d} of the corresponding drive groove 8. Seen in a radial direction, the bottom line Bg of the guide groove 10 is farther away from the screw axis S than the bottom line B_{d} of the drive groove 8. Hence, the guide groove 10 is at an axial position closest to the upper end 7a of the first recess deeper in the radial direction than the corresponding drive groove 8. Due to the bevelled surface of the second recess 9, the depth of the guide grooves 10 gradually increases from the free end 5 towards the guide groove 8. This results in a precise guiding of an engagement protrusion of the screw driver 20 into the first recess 7 while simultaneously facilitating the engagement of the engagement protrusion with the guide groove at the outermost portion at the free end 5 of the guide groove 10.

In a top view, the guide grooves 10 have a greater width than the drive grooves 8. The width of the guide grooves 10 perpendicular to the bottom line Bg decreases in a direction towards the free end 5.

The guide grooves 10 go over in the drive grooves 8 through an intermediate section 11 with a bevelled wall 11 a that conically narrows towards the drive grooves 8. The intermediate section 11 may have a considerably smaller axial height than the first recess 7 and also than the second recess 9. By means of this design, the intermediate section 11 and the guide grooves 10 form pocket-like recesses that allow to catch and guide the engagement protrusions of the screw driver 20 into the guide grooves 8.

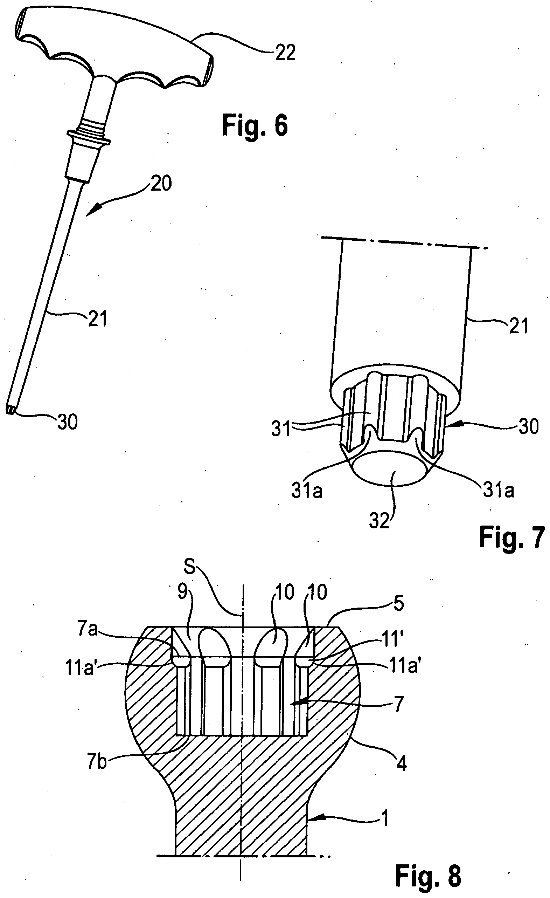

As depicted in Figs. 6 and 7, the screw driver 20 comprises a drive shaft 21, a handle 22 at one end of the drive shaft 21 and the engagement portion 30 at the opposite end. The engagement portion 30 has a substantially cylindrical main contour that fits into the first recess 7 and longitudinally extending rib-like engagement protrusions 31 that are sized so as to engage the drive grooves 8 of the first recess 7 in order to apply torque onto the screw element 1. The engagement portion 30 is bevelled towards the free end surface 32 of the engagement portion that is substantially circular. In addition, the engagement protrusions 31 each have a bevelled front end surface 31a. The length of the bevelled front end surface 31a of the engagement projections 31 corresponds substantially to the length of the conical surface of the second recess 9 between the free end 5 and the intermediate portion 11 of the drive portion 6 of the screw element. An angle of inclination of the bevelled surface 31 may be the same angle as that of the conical recess 9 or that of the slanted wall 11 a of the intermediate portion 11. Such an enlarged bevelled surface contributes to easily locate the drive portion 6 of the screw element even in the case of a restricted view or no view to the operation site.

A modified embodiment of the screw element with a modification of the drive portion 6 is shown in Fig. 8. All parts and portions that are identical to the first embodiment are marked with the same reference numerals and the description thereof is not repeated. The modified embodiment differs in the shape of the intermediate portion. The intermediate portion 11' comprises a rounded wall 11a'.

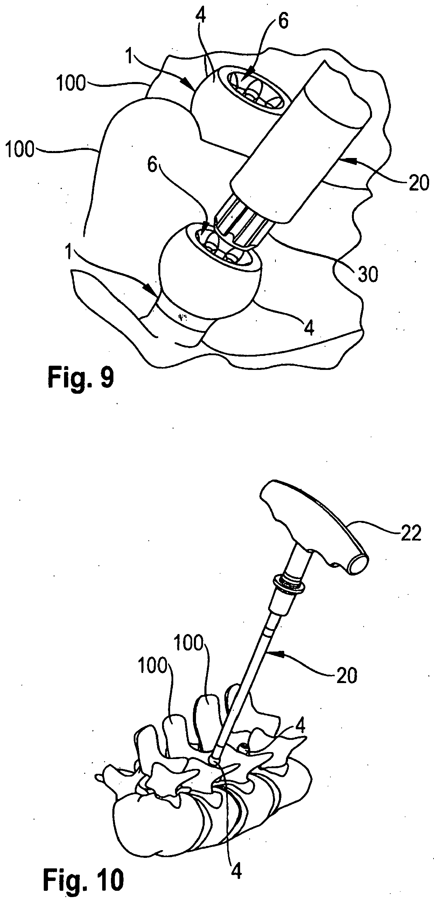

Referring now to Figs. 9 and 10 the application of the screw element and the screw driver according to the first embodiment will be explained. In Fig. 9, two screw elements 1 have already been inserted into the pedicles of two vertebrae 100. Each of the screw elements 1 comprises the drive portion 6 as described before. The insertion depths of the screw elements 1 need to be further adjusted. This is performed with the screw driver 20 that engages with the engagement portion 30 the corresponding drive portion 6 of the screw element 1. Due to the design of the drive portion 6 and the engagement portion 30 of the screw driver, the engagement of the engagement portion 30 with the drive portion is achieved quickly and easily, even if there is no or only a restricted view to the operation site. Therefore, it is possible to carry out the adjustment of a number of pedicle screws in a short time.

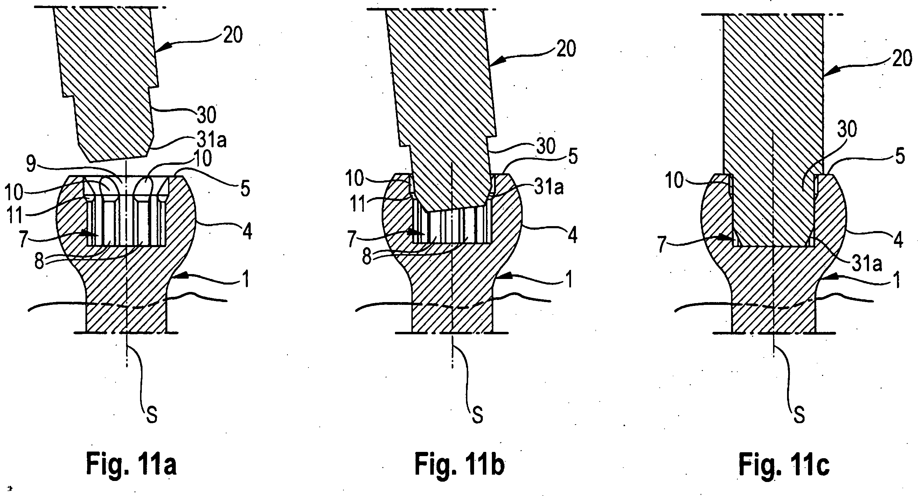

Referring now to Figs. 11a) to 11c) the function of the engagement portion 30 of the screw driver 20 and the drive portion 6 of the screw element is shown in more detail. As depicted in Fig. 11a), it may happen, that the screw driver 20 is approaching the screw element in an inclined manner with respect to the screw axis S. As further shown in Fig. 11b) the engagement portion 30 of the screw driver 20 first engages the conical second recess 9. When the engagement protrusions 31 of the engagement portion 30 of the screw driver 20 begin to engage the guide grooves 10, the screw driver 20 is automatically aligned while penetrating further into the drive portion 6. The guide grooves 10 and the intermediate portion 11 guide the engagement portion 30 into the drive grooves 8 so that the screw element 1 and the screw driver 20 become aligned and connected in a form-fit manner to each other. Then, torque can be applied with the screw driver 20 onto the screw element 1. It shall be noted that due to the decreasing depth and width of the guide grooves 10 towards the free end 5, the engagement portion 30 can be easily rotated until the engagement protrusions locate and engage the engagement grooves 10.

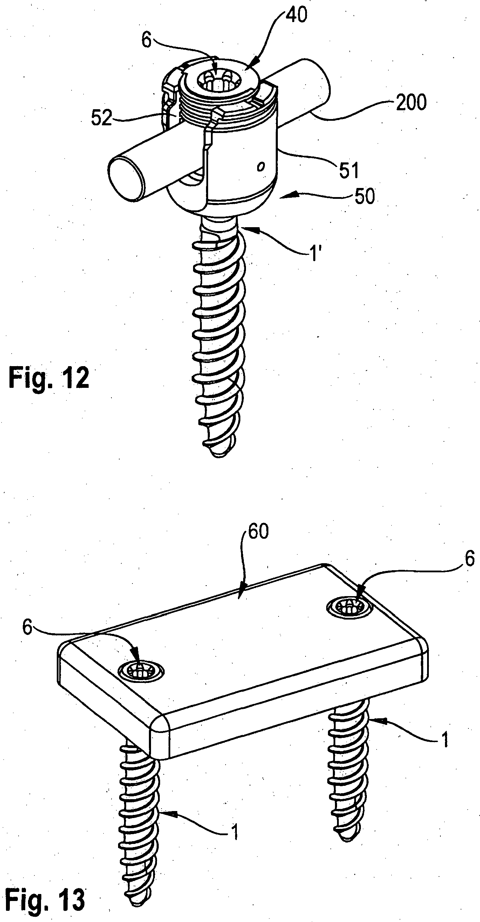

A second embodiment of a screw element will be explained with reference to Fig. 12. Parts and portions that are identical to the previous embodiments have the same reference numerals and the description thereof is not repeated. In this embodiment, the screw element is a set screw 40 that is used in a polyaxial bone anchoring device 50. The polyaxial bone anchoring device 50 is shown only in an exemplary manner, many different designs of such polyaxial bone anchoring device may be contemplated. The polyaxial bone anchoring device 50 comprises a screw element 1' that has a spherical segment-shaped head (not shown) and that may have a known drive portion, such as, for example, a known torx-shaped drive portion or a polygon-shaped drive portion or that may have also a drive portion 6 according to the previous embodiments. The screw element 1' is pivotably connected to a receiving part 51 that comprises a seat to hold the head of the screw element in a ball and socket manner. A pressure element (not shown) may be provided to exert pressure onto the head. The receiving part 51 comprises a substantially U-shaped recess 52 that is configured to receive a rod 200 therein. The rod is intended to be connected to a plurality of bone anchors. To lock the rod 200 in the receiving part 51 and also to lock the head in its pivot position relative to the receiving part 51, a locking element in the form of a set screw 40 is used that cooperates with a thread provided in the receiving part 51. It may be the case, that once the head and the rod are locked, further adjustments become necessary. In such a case the set screw 40 has to be loosened and tightened again after correcting the angular position of the receiving part relative to the head or after correcting the position of the rod. For such adjustments, the screw driver 20 that cooperates with the drive portion 6 in the set screw 40 may be used. Hence, the adjustments can be performed quickly and easily.

It should be noted, that such a type of set screw having the engagement portion 6 could also be used for a monoaxial bone anchor in which the screw element and the receiving part are fixed relative to each other.

A further application is shown in Fig. 13. Fig 13 depicts a bone plate 60 that is used with screw elements 1, for example, in orthopedic and trauma surgery to immobilize broken bone parts. The screw elements 1 are of the type according to Figs. 1 to 5 and 8 and comprise the drive portion 6 in their head. The head may be spherical segment-shaped so that the screw element 1 can be placed within a hole of the bone plate at various angles. Alternatively, the head may have a shape that allows to place the screw element only at a fixed angle with respect to the bone plate 60. Also in such cases, an adjustment of the insertion depth of the bone screw may be necessary. This is facilitated by using the screw element with the drive portion 6 and a corresponding screw driver 20. In a still further modification, a locking element may be provided in the holes of the bone plate to prevent pull-out of the bone screw, wherein the locking element comprises the drive portion 6.

Further embodiments and modifications of the previously described embodiments may be contemplated. The size and the angle of the bevelled surface of the second recess 9 and of the guide grooves 8, as well as of the intermediate portion 11, 11' can be varied. The wall 11a, 11a' of the intermediate portion 11, 11' may have any shape that is configured to guide the engagement portion of the screw driver into the first recess 7.

In the embodiment shown, an even number of drive grooves is shown and each drive groove is positioned opposite to another drive groove. However, also an odd number of drive grooves may be contemplated and one drive groove may not be opposite to another drive groove. The same applies to the corresponding guide grooves.

Instead of the torx-shape of the drive grooves, also a polygonal shape of the first recess may be contemplated. In such a case, the corners of the polygon are considered as the drive grooves.

The bottom lines of the drive grooves and the guide grooves may also be twisted around the screw axis. Also, the bottom lines need not to be exactly parallel to the screw axis.

## Claims

1. A screw element for use in spinal, orthopedic or trauma surgery, the screw element including
a screw axis (S) and a screw thread (3);
a drive portion (6) for engagement with a screw driver (20), wherein the drive portion (6) comprises a first recess (7) with a wall and substantially longitudinally extending drive grooves (8) formed in the wall,
a second recess (9) between a free end (5) of the screw element (1, 1') and the first recess (7), wherein the second recess (9) has a wall and a gradually increasing inner diameter towards the free end (5);
wherein in the wall of the second recess (9) guide grooves (10) are formed at positions corresponding to the position of the drive grooves (8) that allow an engagement portion (30) of a screw driver (20) to be guided to engage the drive grooves (8),
wherein the guide grooves (10) comprise a bottom line (B_{g}) that is further away from the screw axis (S) than a bottom line (B_{d}) of the drive grooves (8).

2. The screw element of claim 1, wherein the first recess (7) is substantially cylindrical and the drive grooves (8) extend substantially parallel to the screw axis.

3. The screw element of claim 1 or 2, wherein the first recess (7) with the drive grooves (8) has a torx-shape.

4. The screw element of one of claims 1 to 3, wherein the second recess (9) is substantially conical with increasing diameter towards the free end (5) of the screw element.

5. The screw element of claim 4, wherein an axial length of the second recess (9) is between one fifth and a half of the axial length of the first recess, preferably between one fourth and one third of the axial length of the first recess (7).

6. The screw element of claim 5, wherein a depth of the guide grooves (10) increases in a direction from the free end (5) towards the first recess (7).

7. The screw element of one of claims 1 to 6, wherein a width of the guide grooves (10) increases in a direction from the free end (5) towards the first recess (7).

8. The screw element of one of claims 1 to 7, wherein a width of the guide grooves (10) is greater than a width of the drive grooves (8) at a position closest to the first recess (7).

9. The screw element of one of claims 1 to 6, wherein an inclined shoulder (11) is provided at an end of each guide groove (10) adjacent to the drive groove (8).

10. The screw element of one of claims 1 to 8, wherein the screw element (1) is a bone screw comprising a shank (2) with a bone thread in at least a portion thereof and with a head (4) having a free end (5) opposite to the shank (2) and wherein the drive portion (6) is provided in the head (4).

11. The screw element of claim 10, wherein the head (4) has a spherical segment-shaped outer surface portion.

12. The screw element of one of claims 1 to 12, wherein the screw element is a set screw (40) configured to be used as a locking element for a polyaxial bone screw (50) or a locking element for a bone plate.

13. A system comprising a screw element according to claims 1 to 12 and a screw driver, the screw driver (20) comprising a drive axis and engagement portion (30) with engagement protrusions extending in a longitudinal direction parallel to the drive axis and configured to engage the drive grooves (8) of the screw element (1),
wherein the engagement protrusions (31) comprise a bevelled front end (31a) with the length of the bevel corresponding substantially to the length of the guide grooves (10).

14. The system of claim 13, wherein the engagement protrusions have a torx-shape in a cross-sectional view perpendicular to the drive axis.

## Patentansprüche

1. Ein Schraubenelement zur Verwendung in der Wirbelsäulen-, orthopädischen oder Trauma-Chirurgie, wobei das Schraubenelement aufweist:
eine Schraubenachse (S) und ein Schraubengewinde (3);
einen Antriebsabschnitt (6) zum Eingriff durch einen Schraubendreher (20), wobei der Antriebsabschnitt (6) eine erste Ausnehmung (7) mit einer Wand und sich im Wesentlichen in Längsrichtung erstreckende Antriebsnuten (8) umfasst, die in der Wand ausgebildet sind,
eine zweite Ausnehmung (9) zwischen einem freien Ende (5) des Schraubenelements (1, 1') und der ersten Ausnehmung (7), wobei die zweite Ausnehmung (9) eine Wand besitzt und einen in Richtung auf das freie Ende (5) allmählich zunehmenden Innendurchmesser besitzt;
wobei in der Wand der zweiten Ausnehmung (9) Führungsnuten (10) an Positionen ausgebildet sind, die den Positionen der Antriebsnuten (8) entsprechen und die es einem Eingriffsabschnitt (30) eines Schraubendrehers (20) ermöglichen, so geführt zu werden, dass sie in die Antriebsnuten (8) eingreifen,
wobei die Führungsnuten (10) eine Grundlinie (B_{g}) umfassen, die weiter von der Schraubenachse (S) entfernt ist als eine Grundlinie (B_{d}) der Antriebsnuten (8).

2. Das Schraubenelement gemäß Anspruch 1, wobei die erste Ausnehmung (7) im Wesentlichen zylindrisch ist, und die Antriebsnuten (8) sich im Wesentlichen parallel zu der Schraubenachse erstrecken.

3. Das Schraubenelement gemäß Anspruch 1 oder 2, wobei die erste Ausnehmung (7) mit den Antriebsnuten (8) eine Torx-Form besitzt.

4. Das Schraubenelement gemäß einem der Ansprüche 1 bis 3, wobei die zweite Ausnehmung (9) im Wesentlichen konisch mit einem in Richtung auf das freie Ende (5) zunehmenden Durchmesser des Schraubenelements ist.

5. Das Schraubenelement gemäß Anspruch 4, wobei eine axiale Länge der zweiten Ausnehmung (9) zwischen einem Fünftel und einer Hälfte der axialen Länge der ersten Ausnehmung beträgt, vorzugsweise zwischen einem Viertel und einem Drittel der axialen Länge der ersten Ausnehmung (7).

6. Das Schraubenelement gemäß Anspruch 5, wobei eine Tiefe der Führungsnuten (10) in einer Richtung von dem freien Ende (5) in Richtung auf die erste Ausnehmung (7) zunimmt.

7. Das Schraubenelement gemäß einem der Ansprüche 1 bis 6, wobei eine Breite der Führungsnuten (10) in einer Richtung von dem freien Ende (5) in Richtung auf die erste Ausnehmung (7) zunimmt.

8. Das Schraubenelement gemäß einem der Ansprüche 1 bis 7, wobei eine Breite der Führungsnuten (10) größer ist als eine Breite der Antriebsnuten (8) an einer Position, die der ersten Ausnehmung (7) am nächsten ist.

9. Das Schraubenelement gemäß einem der Ansprüche 1 bis 6, wobei eine geneigte Schulter (11) an einem Ende jeder Führungsnut (10) benachbart zu der Antriebsnut (8) bereitgestellt ist.

10. Das Schraubenelement gemäß einem der Ansprüche 1 bis 8, wobei das Schraubenelement (1) eine Knochenschraube umfassend einen Schaft (2) mit einem Knochengewinde in zumindest einem Abschnitt desselben und einen Kopf (4) mit einem freien Ende (5) gegenüberliegend dem Schaft (2) umfasst, und wobei der Antriebsabschnitt (6) in dem Kopf (4) bereitgestellt ist.

11. Das Schraubenelement gemäß Anspruch 10, wobei der Kopf (4) einen äußeren Oberflächenabschnitt in der Form eines sphärischen Segments besitzt.

12. Das Schraubenelement gemäß einem der Ansprüche 1 bis 12, wobei das Schraubenelement eine Setzschraube (40) ist, die eingerichtet ist, als ein Verschlusselement für eine polyaxiale Knochenschraube (50) oder als ein Verschlusselement für eine Knochenplatte verwendet zu werden.

13. Ein System umfassend ein Schraubenelement gemäß einem der Ansprüche 1 bis 12 und einen Schraubendreher, wobei der Schraubendreher (20) eine Antriebsachse und einen Eingriffsabschnitt (30) mit sich in Längsrichtung parallel zur Antriebsachse erstreckenden Eingriffsvorsprüngen umfasst, und der eingerichtet ist, in die Antriebsnuten (8) des Schraubenelements (1) einzugreifen,
wobei die Eingriffsvorsprünge (31) ein abgeschrägtes vorderes Ende (31a) mit einer Länge der Abschrägung entsprechend im Wesentlichen der Länge der Antriebsnuten (10) umfassen.

14. Das System gemäß Anspruch 13, wobei die Eingriffsvorsprünge in einer Querschnittsansicht senkrecht zur Antriebsachse eine Torx-Form besitzen.

## Revendications

1. Un élément formant vis destiné à être utilisé dans le cadre d'une chirurgie spinale, orthopédique ou traumatologique, l'élément formant vis comprenant:
un axe de vis (S) et un filetage de vis (3) ;
une partie d'entraînement (6) destinée à venir en prise avec un tournevis (20), la partie d'entraînement (6) comprenant un premier évidement (7) comportant une paroi et des rainures d'entraînement (8) formées dans la paroi et s'étendant de façon substantiellement longitudinale,
un second évidement (9) entre une extrémité libre (5) de l'élément formant vis (1, 1') et le premier évidement (7), le second évidement (9) comportant une paroi et un diamètre intérieur croissant progressivement en direction de l'extrémité libre (5) ;
dans lequel, dans la paroi du second évidement (9), des rainures de guidage (10) sont formées à des positions correspondant à la position des rainures d'entraînement (8) qui permettent le guidage d'une partie de mise en prise (30) d'un tournevis (20) de sorte qu'elle vienne en prise avec les rainures de guidage (8),
dans lequel les rainures de guidage (10) comprennent une ligne inférieure (B_{g}) qui est plus éloignée de l'axe de vis (S) qu'une ligne inférieure (B_{d}) des rainures d'entraînement (8).

2. L'élément formant vis selon la revendication 1, dans lequel le premier évidement (7) est substantiellement cylindrique et les rainures d'entraînement (8) s'étendent de manière substantiellement parallèle par rapport à l'axe de vis.

3. L'élément formant vis selon les revendications 1 ou 2, dans lequel le premier évidement (7) avec les rainures d'entraînement (8) présente une forme hexalobée.

4. L'élément formant vis selon l'une des revendications 1 à 3, dans lequel le second évidement (9) est substantiellement conique avec un diamètre croissant en direction de l'extrémité libre (5) de l'élément formant vis.

5. L'élément formant vis selon la revendication 4, dans lequel une longueur axiale du second évidement (9) mesure entre un cinquième et la moitié de la longueur axiale du premier évidement, de préférence entre un quart et un tiers de la longueur axiale du premier évidement (7).

6. L'élément formant vis selon la revendication 5, dans lequel une profondeur des rainures de guidage (10) augmente dans une direction allant de l'extrémité libre (5) vers le premier évidement (7).

7. L'élément formant vis selon l'une des revendications 1 à 6, dans lequel une largeur des rainures de guidage (10) augmente dans une direction allant de l'extrémité libre (5) vers le premier évidement (7).

8. L'élément formant vis selon l'une des revendications 1 à 7, dans lequel une largeur des rainures de guidage (10) est supérieure à une largeur des rainures d'entraînement (8) au niveau d'une position la plus proche du premier évidement (7).

9. L'élément formant vis selon l'une des revendications 1 à 6, dans lequel un épaulement incliné (11) est prévu au niveau d'une extrémité de chaque rainure de guidage (10) adjacente à la rainure d'entraînement (8).

10. L'élément formant vis selon l'une des revendications 1 à 8, l'élément formant vis (1) étant une vis à os comprenant une tige (2) comportant un filetage à os dans au moins une partie de celle-ci et comportant une tête (4) comprenant une extrémité libre (5) opposée à la tige (2) et dans lequel la partie d'entraînement (6) est prévue dans la tête (4).

11. L'élément formant vis selon la revendication 10, dans lequel la tête (4) comporte une partie de surface extérieure en forme de segment sphérique.

12. L'élément formant vis selon l'une des revendications 1 à 12, l'élément formant vis étant une vis de pression (40) configurée pour être utilisée en tant qu'élément de verrouillage pour une vis à os polyaxiale (50) ou en tant qu'élément de verrouillage pour une plaque osseuse.

13. Un système comprenant un élément formant vis selon les revendications 1 à 12 et un tournevis, le tournevis (20) comprenant un axe d'entraînement et une partie de mise en prise (30) comportant des protubérances de mise en prise s'étendant dans une direction longitudinale parallèle à l'axe d'entraînement et configurées pour venir en prise avec les rainures d'entraînement (8) de l'élément formant vis (1),
dans lequel les protubérances de mise en prise (31) comprennent une extrémité avant biseautée (31a), la longueur du biseau correspondant substantiellement à la longueur des rainures de guidage (10).

14. Le système selon la revendication 13, dans lequel les protubérances de mise en prise présentent une forme hexalobée dans une vue en section transversale perpendiculaire à l'axe d'entraînement.
